# EUROPEAN PATENT SPECIFICATION

(11) **EP 1 835 863 B1**
(45) Date of publication and mention of the grant of the patent: **27.05.2015**
(21) Application number: 06700211.3
(22) Date of filing: 09.01.2006
(51) Int. Cl.: A61B 19/08, A61B 19/00

(54) **DRAPING PRODUCT WITH AN ADHESIVE EDGE, INCISION FILM OR TAPE**
DRAPIERPRODUKT MIT KLEBEKANTE, EINSCHNITTFOLIE ODER -BAND
PRODUIT DRAPANT AVEC UN BORD ADHESIF, FILM D'INCISION OU BANDE

(30) Priority: 11.01.2005 SE 0500059
(43) Date of publication of application: 26.09.2007
(73) Proprietor: Mölnlycke Health Care AB, 402 52 Göteborg (SE)
(72) Inventor: JOHANSSON, Helena, 423 38 Torslanda (SE); LAGER, Katarina, 433 61 Sävedalen (SE)
(74) Representative: Zacco Sweden AB
(86) International application number: PCT/SE2006/000021
(87) International publication number: WO 2006/075946

(56) References cited:
- WO-A1-89/02259
- WO-A1-96/09076
- WO-A1-03/079920
- US-A- 6 117 111

## Description

### TECHNICAL FIELD

The present invention relates to a draping product, an incision film or tape, which on its under side is coated fully or partially with adhesive.

### BACKGROUND ART

Draping products, such as surgical drapes and surgical sheets, with an adhesive edge are often applied around an operation site in order to provide a barrier between the operation site and that part of the patient's body which lies outside the operation site. This barrier is intended, on the one hand, to prevent bacteria and the like from the patient's body from contaminating the operation site and, on the other hand, to prevent blood, bacteria and similar from the operation site from finding their way onto the patient's body in those areas which lie outside the operation site or from contaminating the operating table and other operating theatre equipment. The edge of a surgical drape or a surgical sheet which extends adjacent to the operation site is thus required to adhere tightly to the skin, and the inherent strength of the adhesive attachment must be so great that the draping product remains securely in place under the loadings to which it is normally subjected during an operation. In order for this barrier to function in a satisfactory fashion, it is important for the adhesive edge to provide a tight seal and for it to be impermeable to fluids.

Many modern draping materials possess, as previously mentioned, an integrated self-adhesive edge to prevent micro organisms from coming into contact with the operation site. In order to avoid contamination, and thus infection, it is of the greatest importance for a completely tight seal to be present between the self-adhesive edge and the skin. In the event that the self-adhesive edge does not provide a completely tight seal, it is possible for bacteria that are present on the skin beneath the draping product and have not been treated with a disinfectant to be transported by the heated air beneath the draping material and to pass through the space between the edge and the skin and, in so doing, to contaminate the operation wound via the air.

Bacteria can naturally also be transported by fluid, which results in a considerably larger quantity compared with dry contact.

The self-adhesive edge adjacent to an operation wound must, therefore, adhere extremely tightly to the skin in order to form a secure barrier against bacteria that are transported by fluid or air.

The adhesion to the skin must also be so great that the product remains securely in place and withstands the external loadings to which the draping product is subjected.

Given that the skin is not a smooth surface, but exhibits cracks, folds and other unevenness in the skin, which can vary in depth from a few micrometres up to several hundred micrometres, it is not possible to seal all skin folds and cracks with the adhesives and thicknesses that are used today on self-adhesive edges. This can result in bacteria being transported to the wound under the self-adhesive edge.

The thickness of the skin folds varies between different points on the body, and between different persons, ages and skin types, etc. These are common variations in the topography of the skin and are encountered on all skin types. The skin is smoother in younger persons, however, whereas older skin has lost a proportion of its elasticity and the skin folds are deeper. Dry skin also often has deeper cracks than moist skin. Shearing forces acting on the adhesive edge during the operation can give rise to can a separation between the layers of skin. This leads to the formation of a blister or a bruise, that is to say an accumulation of blood under the skin. The blisters or bruises that occur along the self-adhesive edge usually located at different depths in the epidermis or at the boundary between the epidermis and the dermis. The shearing forces which act on the edge can arise, on the one hand, as a result of the weight of the draping product, including fluid in bags or fluid absorbed into the product, but also as a result of a swelling occurring during the operation.

Previously disclosed in WO 2003/079919 A1 and WO 2003/079920 A1 is the use of skin friendly adhesives, that is to say adhesives which, when removed from the skin, do not take parts of the stratum corneum with them to any significant degree, for the adhesive edge of surgical drapes and surgical sheets. Such edge adhesives prevent the flow of fluid through the barrier, although it has nevertheless been established that, in the case both of these adhesives and of other adhesives that are customary in this context, there is a risk of fluid leaking through the barrier via skin cracks, skin folds or other unevenness in the skin.

WO 2003/079920 discloses features falling under the preamble of claim 1.

The expression draping product is used in the present application to denote surgical gloves, surgical drapes, surgical drapes with a hole, extremity drapes and slotted drapes, special products in various areas of surgery, such as ophthalmologic surgery, oto-rhino-laryngological surgery, plastic surgery, paediatric surgery, general surgery, orthopaedic surgery, neurological surgery, gynaecological surgery, urological surgery, cardiac and vascular surgery, obstetric surgery and similar products. Draping products can be encountered in the form of individually packaged products or sets comprising a plurality of products. The products are also packed in sterile conditions.

An incision film consisting of a transparent plastic film coated with adhesive is often applied to the operation site itself. The incision film should also be attached to the skin of a patient in a sealing fashion. The same thing naturally also applies to surgical tapes, which are used, for example, to attach tubes to the skin of a patient.

The object of the present invention is to eliminate or at least significantly to reduce the risk of leakage through adhesive fluid barriers in draping products, incision films and tapes.

### DISCLOSURE OF INVENTION

This object is achieved in accordance with the invention by means of a draping product with an adhesive edge, an incision film or a tape as defined in the appended claims.

In a preferred embodiment, the draping product with an adhesive edge, the incision film or the tape is leakproof in accordance with the MHC Leakage Test with a groove depth of 75 micrometres, 150 micrometres and 200 micrometres. The adhesive has a weight per unit area of 80 g/m² or more and a softness greater than 10 mm. The adhesive preferably has a weight per unit area of 200 g/m² or more and a softness greater than 10 mm.

In a special embodiment, the draping product with an adhesive edge, the incision film or the tape, in conjunction with which the adhesive of the draping product is attached to a plastic film projecting from one edge of the product, is characterized in that the adhesive has a weight per unit area of 50 g/m² or more and a softness greater than 10 mm.

The adhesive may with advantage possess a softness greater than 12 mm, preferably greater than 14 mm, and preferably consists of a hot-melt adhesive or a silicon elastomer.

### BRIEF DESCRIPTION OF DRAWINGS

The invention is now described below with reference to the accompanying Figures, in which:
Fig. 1 illustrates schematically a plan view from above of a draping system in accordance with a preferred embodiment of the invention;
Fig. 2 illustrates a sectioned view along the line II-II in Figure 1;
Figs. 3-7 illustrate plan views of various draping products;
Fig. 8 illustrates schematically the measurement of the strength of the adhesive attachment to the skin;
Fig. 9 illustrates a cone used for measurement of the softness;
Fig. 10 illustrates a method of measurement for measuring the softness;
Figs. 11-13 illustrate the result of the MHC Leakage Test for various products;
Fig. 14 illustrates the result of the MHC Leakage Test for a film coated with adhesive with different weights per unit area and softness, and
Figs. 15-21 illustrate the MHC Leakage Test.

### MODE(S) FOR CARRYING OUT THE INVENTION

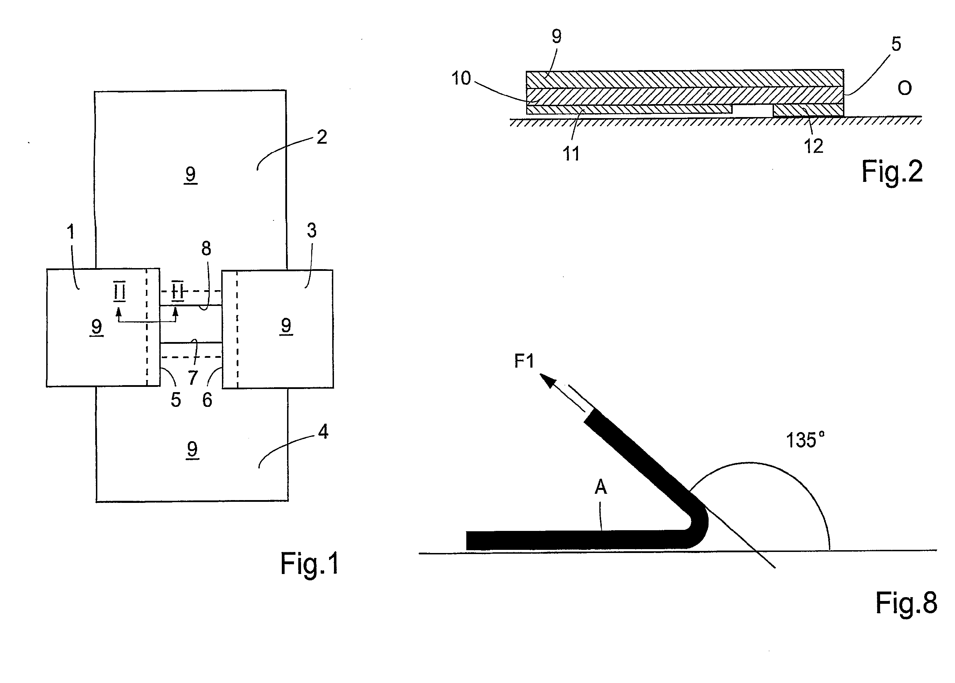

Illustrated schematically in Figure 1 is a draping system comprising four draping products 1-4 applied around an operation site O on a patient, not shown here. The draping products 1 and 3 demarcate two opposing parallel edges 5, 6 of the operation site, and the draping products 2 and 4 demarcate two opposing parallel edges 7,8, which are perpendicular to the edges 5,6. In order to prevent fluid from the operation site from being able to leak under the edges 5-8 or bacteria from the area outside the operation site from being able to find their way into the operation site, the edges 5-8 are attached to the patient's skin by means of an adhesive. The draping products 1-4 can consist with advantage of surgical drapes and surgical sheets with the designation Klinidrape® supplied by Mölnlycke Health Care AB, Sweden, which consist of a laminate having three layers, a fluid-absorbent top layer 9 of nonwoven fabric material (a so-called nonwoven), a fluid-impermeable intermediate layer of polyethylene 10 and, at the bottom, an absorbent layer 11 of cellular or alternatively nonwoven material. The purpose of the top layer is to absorb blood and other fluids that are released from the operation site in order to prevent contamination of the patient by the surgical personnel and contamination of the surgical personnel and the operating theatre. The plastic film constitutes a barrier against the fluid-borne transport of bacteria between the patient and the operation site, and the layer of cellular material is intended to increase the comfort for the patient by absorbing perspiration and preventing direct contact between the patient's skin and the plastic film. The draping products 1-4 also have an adhesive coating 12 along their edges 5-8.

The draping products 1-4 can also consist with advantage of surgical drapes and surgical sheets marketed under the name BARRIER™ from Mölnlycke Health Care AB, Sweden.

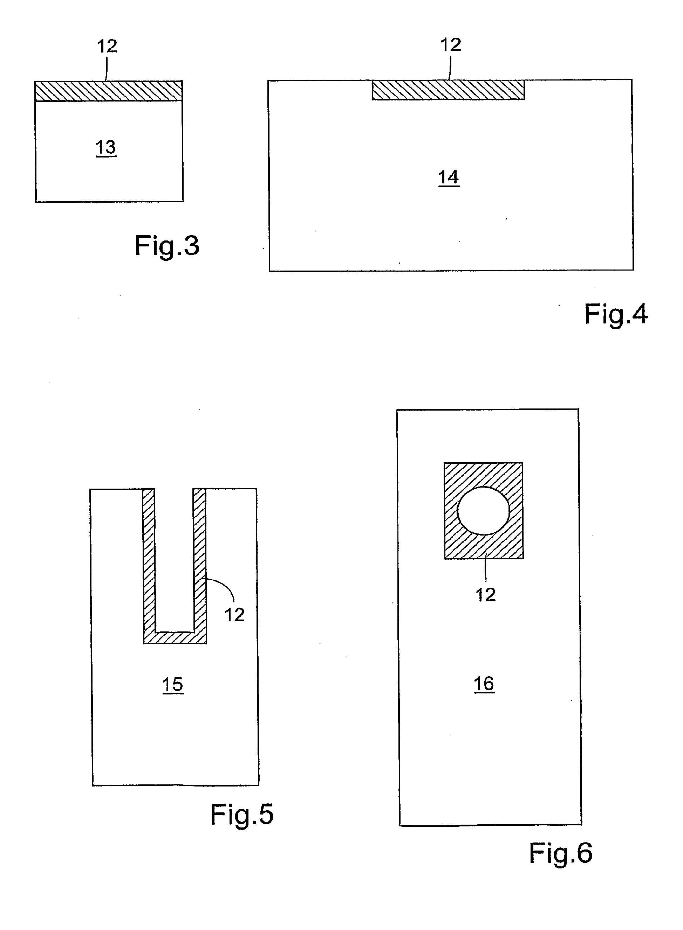

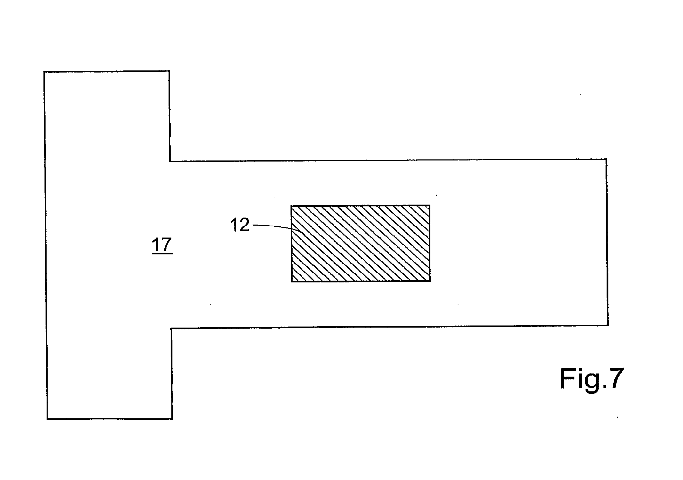

There is a plurality of different embodiments of draping products. The hole size and the form of the hole can vary depending on the type of surgical intervention. Likewise, one or more holes may be present in the same draping product Incision film can be integrated into the draping product or may be provided separately. Similarly, the sizes and types of materials that are used in the draping products may vary. Illustrated in Figures 3-7 are examples of different draping products 13-17 provided with adhesive coatings 12. Figure 3 illustrates a surgical towel, Figure 4 a surgical drape 14, Figure 5 a slotted drape (split sheet), Figure 6 a drape with a hole and Figure 7 a special drape with an adhesive-coated incision film.

The principal function of the adhesive coating 12 is to attach the draping product tightly to the patient's skin, so that fluid-borne transport of bacteria between the patient and the operation site is prevented, and to attach the draping product securely to the patient in such a way that the product remains in place during all the normal loadings to which the draping product is subjected during an operation. It should be pointed out in this context that those draping products which extend in the longitudinal sense of the patient normally hang down from the operating table and as such are very often subjected to the highest loading. The weight of the fluid that is absorbed or taken up in some other way by the draping product, for example by means of bags executed in or attached to the draping product, will also impose a loading on the attachment of the draping product. The adhesive coating is dimensioned in order reliably to withstand the maximum loading to which the attachment of the draping product is normally subjected.

Furthermore, the adhesive in the coating must be skin friendly and must permit the removal of the draping product without causing damage to the skin around the operation site. This requirement presents a major problem in the case of those types of pressure-sensitive adhesive that are currently used as adhesive coatings for draping products. Such adhesives often attach themselves to the skin so strongly that parts of the Stratum Corneum, that is to say the uppermost layer of the skin, become stuck to the adhesive and are pulled away from the skin when the attachment of the draping product is released. This can lead to irritation of and damage to the skin, especially for patients with a sensitive skin, for example patients aged over 70 years, children aged under 3 years, and patients with certain illnesses, such as eczema, or undergoing certain treatments, such as cortisone treatment. In the case of such patients, it is occasionally necessary to apply surgical draping products without using the adhesive edge that is normally present on the draping product, and to attach the draping product by some other means, for example by attaching the draping product with the help of a plurality of pieces of securing tape.

The adhesive attachment of the draping product is subjected essentially exclusively to shearing forces during use of the draping product, which means that the strength of the attachment can be increased by increasing the surface area of the adhesive coating, that is to say the width of the adhesive coating along the edge of the draping product.

Because the characteristics of the skin vary from person to person, the adhesion capacity to the skin of the adhesive coating naturally also varies for different patients. The values for the strength of the adhesive attachment to the skin of an adhesive, as indicated below, must be measured by means of a method of the kind illustrated schematically in Figure 8. Strips A of the adhesive-coated material to be tested with a width of 25 mm are applied to the skin on the back of at least ten healthy persons of varying ages and sex and are left in place on the skin for 6 hours. The strips A are then pulled off at a rate of 25 mm/sec, and the removal force F1 is measured. The angle of removal, that is to say the obtuse angle that is formed between the surface of the skin and the removed part of the strip A, must be 135°. The strength of the adhesive attachment to the skin of the tested adhesive is constituted by the mean value of the force Fl. Adhesives suitable for use in draping products in accordance with the disclosure must exhibit an adhesive strength of at least 0.5 N/25 mm.

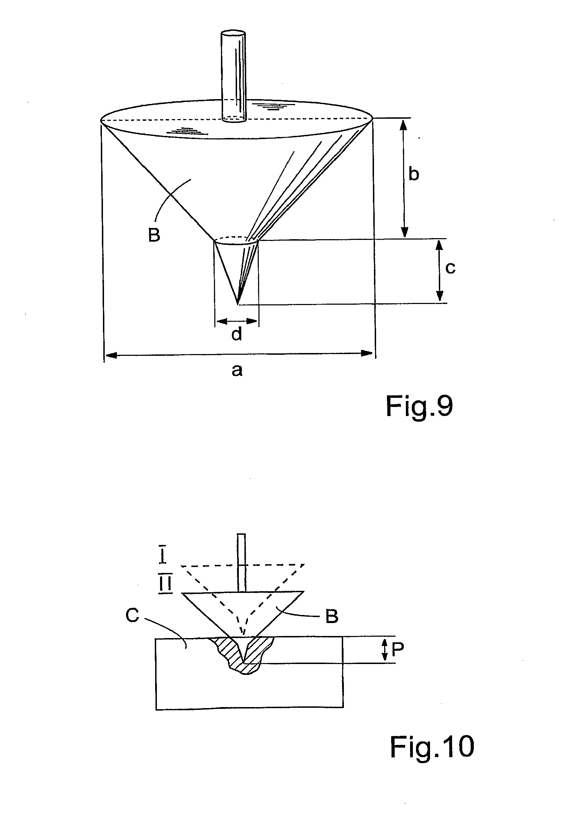

Adhesives that are suitable for use in accordance with the present invention must exhibit a softness that exceeds 10 mm measured by means of a method based on ASTM D 937 and DIN 51580. Certain deviations, as can be appreciated below, have been made. Figures 9 and 10 illustrate this modified method of measuring the softness of an adhesive by causing a cone B with a weight of 62.5 g to penetrate down by the effect of gravity into a 30 mm thick test piece C of the adhesive of which the softness is to be determined. The test piece is obtained by filling a cylindrical glass container having an internal diameter of 60 mm and an internal height of 35-40 mm, with adhesive to a depth of 30 mm. The cone used is illustrated in Figure 9 and has the following dimensions: a=65 mm, b=30 mm, c=15 mm and d=8.5 mm. In the performance of the method for measurement of the softness, the cone B is first lowered down into a position I, as illustrated with broken lines in Figure 10, and in which the tip of the cone just touches the surface of the test piece C. The cone B is then released, so that it is able to penetrate down into the test piece C by the effect of gravity. The number of millimetres by which the tip B of the cone C has penetrated into the test piece C after 5 seconds is measured and constitutes the penetration value P, the value of which is greater in proportion to the softness of the test piece. The penetration value P represents the softness index used in the present invention. A PNR 10 penetrometer supplied by Sommer & Runge KG, Germany is used in the performance of the method.

It has also been found that, in the case of soft, skin friendly adhesives, which form barriers preventing fluid from flowing through them, fluid is capable of leaking through these barriers via cracks in the skin, folds in the skin or other unevenness in the skin. This leakage can give rise to the propagation of bacteria, which in turn can lead to wound infections.

Surprisingly, it has also been found that the above-mentioned risk of leakage can be eliminated, or at least significantly reduced, for a soft, skin friendly adhesive if the weight per unit area of the adhesive and and/or its softness are increased.

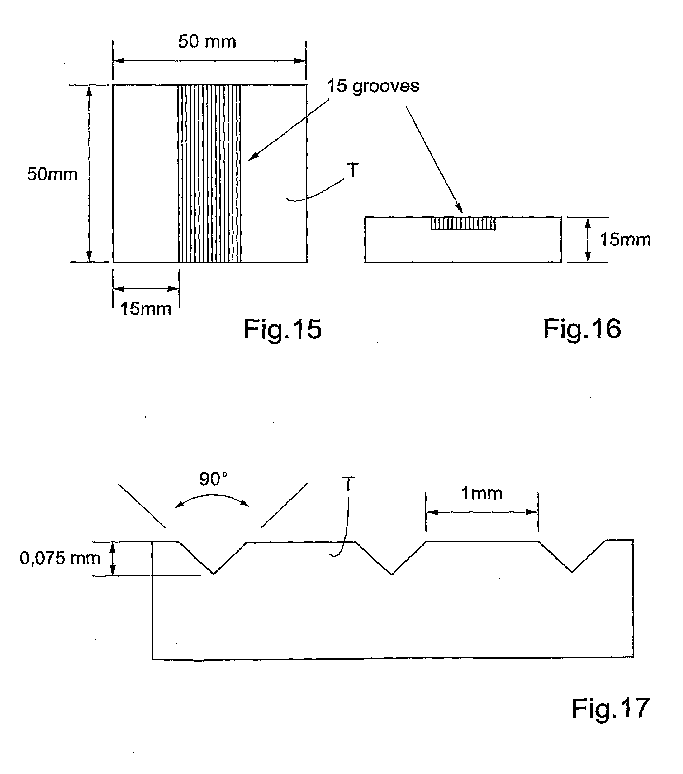

The method known as the MHC Leakage Test described below was developed by the applicants for the purpose of determining whether or not a coating of a soft, skin friendly adhesive is leakproof. Test pieces with a size of 30 x 30 mm from the product to be tested are taken, and a circular hole (d = 12 mm) is removed from the centre of the samples by punching. A coloured test fluid is prepared by mixing 0.2% by weight of Patentblått V (from VWR International, Sweden) and 0.1% by weight of Teepol Gold (from Teepol Products, UK) with de-ionized water. An aluminium test plate having dimensions of 15 x 50 x 50 mm and provided with 15 milled grooves is made; see Fig. 15 (viewed from above) and Fig. 16 (viewed from the side). For a more detailed description of the form of the grooves, see Fig. 17 (section through the plate, viewed from the side). Illustrated in Figure 17 are grooves with a depth of 75 micrometres, although other groove depths can be used in the test depending on what depth of cracks or folds in the skin the product is intended to seal.

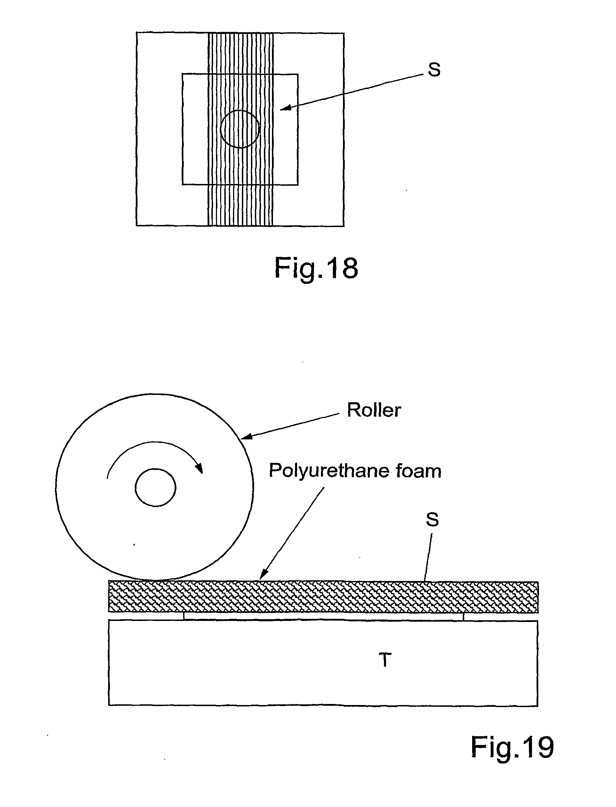

A specimen is then carefully positioned centrally above the grooves of the test piece in such a way that no air bubbles are produced between the test plate and the specimen; see Fig. 18. No pressure may be exerted on the sample when it is positioned against the plate, so that, in the event that air bubbles are produced, these must not be forced away with the help of the fingers, but the sample must be raised and repositioned, or scrapped.

A piece of polyurethane foam (L00562-6, 1.6 mm from Rynel, Inc., Boothbay, ME, USA) having dimensions of 50 x 50 mm is then placed above the sample and the test plate. A mangle made of metal (44 mm wide, r = 48 mm, weight = 995 g) is then rolled over the foam and the specimen at a speed of 5 mm/second; see Fig. 19. The mangle is rolled back and forth once over the sample.

The piece of foam is removed from the sample, and 65 µl of the test fluid are placed in the hole on the specimen with the help of a pipette. The test fluid is distributed uniformly in the hole with the help of the tip of the pipette, so that the fluid reaches every point on the edge of the sample. A stop watch is started as soon as all the test fluid is uniformly distributed in the hole. After 30 minutes, a picture is taken with a digital camera of the specimen and the test fluid placed on the test plate together with a calibrated ruler.

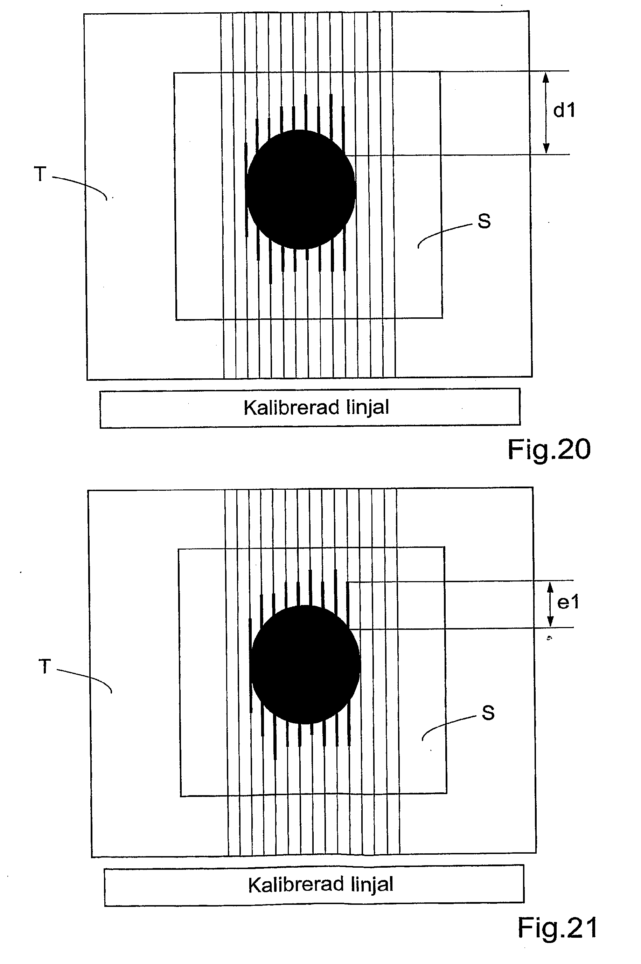

The photograph is used to measure the following distances. For all the grooves that are in contact with the hole on the sample, that is to say in all the grooves into which fluid may be expected to penetrate, the distance d from the edge next to the hole to the edge on the end of the sample is measured, see Fig. 20, which indicates this distance d1 for one of the grooves. All these distances d are then added together, and they constitute the total distance for which it is possible for the sample to leak. After this, the distance e for which the test fluid has leaked in all the grooves on the plate is measured; see Fig. 21, which shows the distance e1 for one of the grooves. The combined length of all the distances e represents the total leakage distance.

Finally, the leakage is obtained by dividing the combined leakage distance by the total distance for which it is possible for the sample to leak. This quotient is then converted into a percentage by multiplying it by 100. The evaluation of the sealing is performed as follows: Result > 10% leakage, regarded as leakage. Result ≤ 10% leakage, regarded as sealing.

Note that, between each measurement on the test plate, the plate must be cleaned in the following way. The plate is first rinsed with water, and it is then washed with n-heptane. It is important to ensure that no adhesive residues remain in the grooves on the plate, and a soft material of the nonwoven compress type (Mesoft, Mölnlycke Health Care) can be dipped in n-heptane and used to rub away adhesive residues in the grooves on the plate. Finally, the plate must be left to dry in the air before it can be reused.

Other solvents may be used for adhesives that are not soluble in n-heptane.

If the security against leakage is to be tested for products that are not transparent, a transparent plastic film is coated with the adhesive which the product contains, after which specimens with an area of 30 x 30 mm are punched from this material. The above-mentioned plastic film must be selected so that its bending length corresponds to the bending length of the carrier in the non-transparent product that is to be tested measured by the "Determination of bending length" method, ISO 9073-7:1995. The MHC Leakage Test is then performed, as described above.

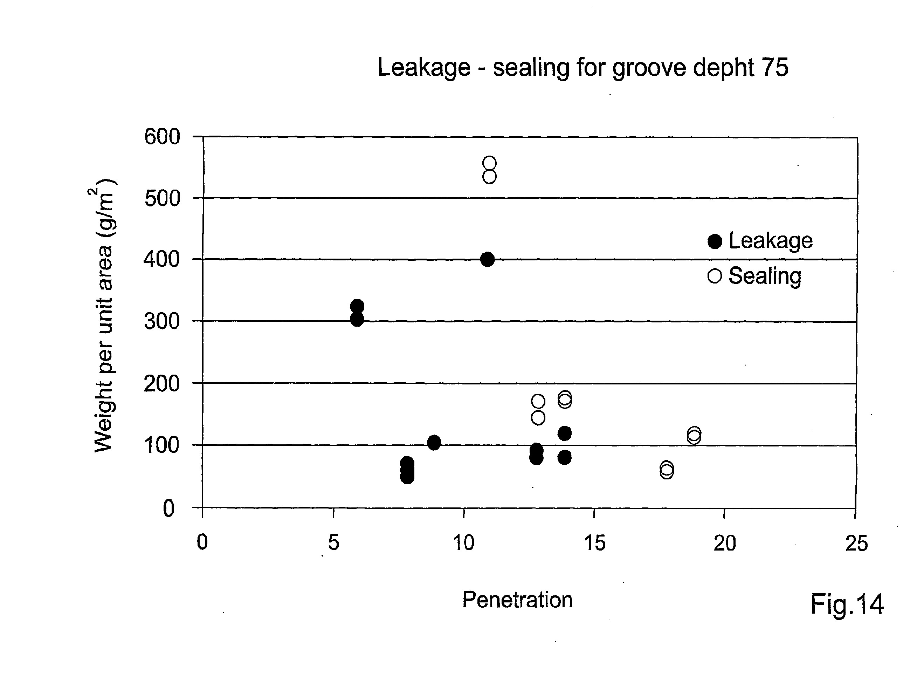

The MHC Leakage Test with a groove depth of 75 micrometres was performed on a polyurethane film with a thickness of 25 ± 5 micrometres, which was coated with a Silgel 612 silicon elastomer supplied by Wacker Chemie GmbH, Germany, with different softness values and weights per unit area. The results are shown in Figure 14.

The results clearly indicate that there is a link between the softness (penetration) and the weight per unit area of the silicon elastomer. The softer the silicon elastomer, the smaller the weight per unit area required for sealing. The result points to the fact that, for a sufficient number of measurements, it is possible to produce a curve that indicates exactly the minimum weight per unit area that is required at a given softness to ensure sealing against the skin. The results make it clear that such a curve has a steep incline initially, that is to say in the case of less soft adhesives, after which it levels out. It is obvious that, at softness values below 10 mm, it is difficult, and perhaps even impossible, to achieve fluid-tight products with the selected adhesive, whereas, at softness values in the order of 20 mm, a weight per unit area of 50 g/mm² may be sufficient to achieve sealing.

When using other adhesives, it can be expected that the values will change, but that the qualitative appearance of the curve will remain the same.

The carrier is an important part of the product, and this, too, has a major effect on the degree of sealing, especially in the case of low weights per unit area for the adhesive coating. The more sensitive the material, the better the carrier is capable of following the folds in the skin and, as a consequence of this, a smaller weight per unit area is required by a soft adhesive. If the carrier is rigid, the flexibility and the sensitivity must be present to a higher degree in the adhesive bulk, which calls for a greater adhesive bulk with a higher weight per unit area. A rigid carrier thus requires a higher weight per unit area for the adhesive coating than a less rigid carrier in order to produce sealing.

It has also emerged that an increased weight per unit area increases the adhesion in a draping product. External shearing forces can then be absorbed and distributed in the adhesive layer, instead of influencing the attachment between the patient's skin and the adhesive. This reduces the risk that the surgical drape may work loose fully or partially under external loading, thereby contributing to a reduced risk of post-operative infection for the patient.

Shearing forces which act on the self-adhesive edge can also give rise to can a separation between the different layers of skin. This results in the formation of a blister, that is to say a well-defined accumulation of serous fluid. The blisters occur along the self-adhesive edge and are usually located at different depths in the epidermis or at the boundary between the epidermis and the dermis. The shearing forces which act on the edge can arise, on the one hand, from external loading, but also as a result of a swelling occurring during the operation. By increasing the weight per unit area on the adhesive, and by utilizing the softest possible adhesive, that is to say a high value for the penetration, the adhesive layer will absorb a large proportion of the shearing forces that would otherwise have acted upon the skin.

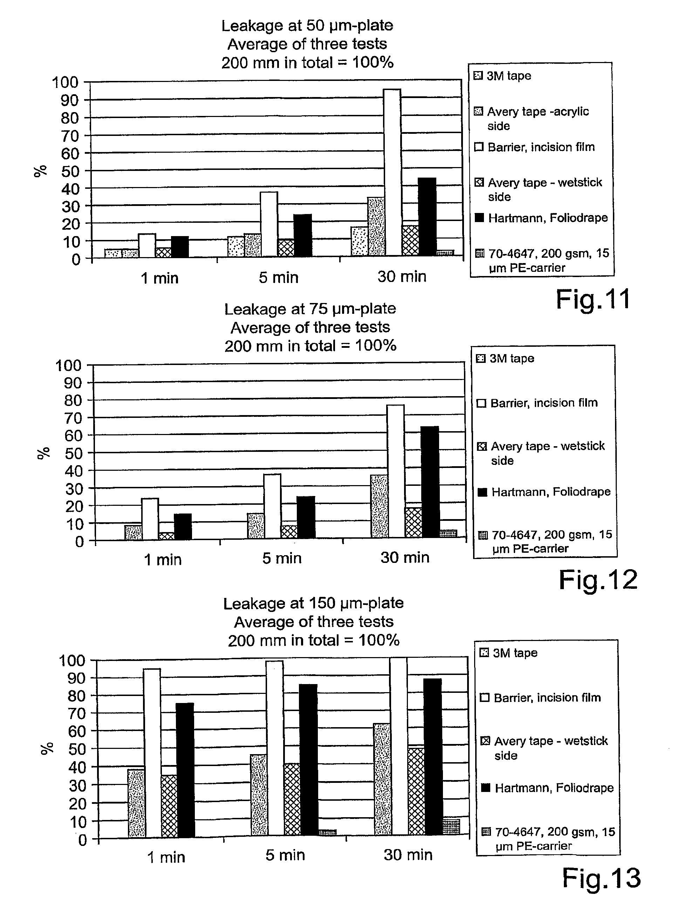

Figures 11-13 illustrate different products tested by means of the MHC Leakage Test with groove depths of 50, 75 and 150 micrometres respectively and illustrate the leakage after 1 minute, 5 minutes and 30 minutes. The tested products were 3M™ Hi-Tack Double Coated Medical Tape, Product Number 1517 supplied by 3M, USA; MED 6370U, Avery Dennison™, Acrylic Adhesive supplied by Avery Dennison, USA; Barrier Flex supplied by Neschen AG, Germany; MED 6370U Avery Dennison™, Wetstick Adhesive supplied by Avery Dennison, USA; Foliodrape®, Hartmann, Transparent OP-tape, No. 258 542, LOT 348 01705, Exp. Date 2008-12 supplied by Hartmann, Germany, and DISPOMELT 70-4647, 200 gsm ±20 gsm supplied by National Starch & Chemical, USA, PE-carrier (15 µm).

As can be appreciated from Figures 11-13, all the samples leaked, apart from the polyethylene film coated with DISPOMELT 70-4647 hot-melt adhesive at all groove depths. The hot-melt adhesive had a softness of 14.7 mm.

Of the tests carried out, it can thus be appreciated that it is possible to produce leakproof products in conjunction with the use of soft adhesives by increasing the weight per unit area of the adhesive coating. The test also shows that the polyethylene film coated with the DISPOMELT 70-4647 hot-melt adhesive is leakproof for the majority of cracks in the skin or folds in the skin that are encountered in normal skin. The products proposed in the present invention are normally supplied packed in sterile conditions, which means that the adhesives used must be capable of being sterilized, as must other components of such articles, of course.

## Claims

1. A draping product (1, 2, 3, 4, 13, 14, 15, 16, 17) with an adhesive edge, an incision film or a tape, provided with a coating (12) of a soft, skin friendly-adhesive, **characterized in that** the soft, skin friendly adhesive has a softness greater than 10 mm measured by means of the modified method of measuring the softness as disclosed in the description, and the draping product with an adhesive edge, the incision film or the tape, provided with a coating (12) of a soft, skin friendly- adhesive, is leakproof in accordance with the MHC Leakage Test with a groove depth of 50 micrometres, the method of said MHC Leakage Test being disclosed in the description.

2. A draping product (1, 2, 3, 4, 13, 14, 15, 16, 17) with an adhesive edge, an incision film or a tape in accordance with Claim 1, **characterized in that** the draping product (1, 2, 3, 4, 13, 14, 15, 16, 17), the incision film or the tape is leakproof in accordance with the MHC Leakage Test with a groove depth of 75 micrometres.

3. A draping product (1, 2, 3, 4, 13, 14, 15, 16, 17) with an adhesive edge, an incision film or a tape in accordance with Claim 2, **characterized in that** the draping product (1, 2, 3, 4, 13, 14, 15, 16, 17), the incision film or the tape is leakproof in accordance with the MHC Leakage Test with a groove depth of 150 micrometres.

4. A draping product (1, 2, 3, 4, 13, 14, 15, 16, 17) with an adhesive edge, an incision film or a tape in accordance with Claim 3, **characterized in that** the draping product (1, 2, 3, 4, 13, 14, 15, 16, 17), the incision film or the tape is leakproof in accordance with the MHC Leakage Test with a groove depth of 200 micrometres.

5. A draping product (1, 2, 3, 4, 13, 14, 15, 16, 17) with an adhesive edge, an incision film or a tape in accordance with Claim 1, in conjunction with which the adhesive of the draping product (1, 2, 3, 4, 13, 14, 15, 16, 17) is attached to a plastic film projecting from one edge of the product, **characterized in that** the adhesive has a weight per unit area of 50 g/m² or more.

6. A draping product (1, 2, 3, 4, 13, 14, 15, 16, 17) with an adhesive edge, an incision film or a tape in accordance with Claims 1, 2 or 5, **characterized in that** the adhesive has a weight per unit area of 80 g/m² or more.

7. A draping product (1, 2, 3, 4, 13, 14, 15, 16, 17) with an adhesive edge, an incision film or a tape in accordance with Claim 1, **characterized in that** the adhesive has a weight per unit area of 200 g/m² or more.

8. A draping product (1, 2, 3, 4, 13, 14, 15, 16, 17) with an adhesive edge, an incision film or a tape in accordance with one or other of Claims 1-7, **characterized in that** the adhesive has a softness greater than 12 mm.

9. A draping product (1, 2, 3, 4, 13, 14, 15, 16, 17) with an adhesive edge, an incision film or a tape in accordance with one or other of Claims 1-7, **characterized in that** the adhesive has a softness greater than 14 mm.

10. A draping product (1, 2, 3, 4, 13, 14, 15, 16, 17) with an adhesive edge, an incision film or a tape in accordance with one or other of Claims 1-9, **characterized in that** the adhesive consists of a hot-melt adhesive.

11. A draping product (1, 2, 3, 4, 13, 14, 15, 16, 17) with an adhesive edge, an incision film or a tape in accordance with one or other of Claims 1-9, **characterized in that** the adhesive consists of a silicon elastomer.

## Patentansprüche

1. Drapierprodukt (1, 2, 3, 4, 13, 14, 15, 16, 17) mit einer Klebekante, einer Einschnittfolie oder einem Band, versehen mit einer Beschichtung (12) eines weichen, hautfreundlichen Klebemittels, **dadurch gekennzeichnet, dass** das weiche, hautfreundliche Klebemittel eine Weichheit größer als 10 mm aufweist, gemessen mittels des modifizierten Verfahrens zum Messen der Weichheit wie in der Beschreibung offenbart, und das Drapierprodukt mit einer Klebekante, der Einschnittfolie oder dem Band, versehen mit einer Beschichtung (12) eines weichen, hautfreundlichen Klebemittels, gemäß der MHC-Dichtigkeitsprüfung mit einer Rillentiefe von 50 Mikrometern leckdicht ist, welches Verfahren der MHC-Dichtigkeitsprüfung in der Beschreibung offenbart ist.

2. Drapierprodukt (1, 2, 3, 4, 13, 14, 15, 16, 17) mit einer Klebekante, einer Einschnittfolie oder einem Band nach Anspruch 1,
**dadurch gekennzeichnet, dass** das Drapierprodukt (1, 2, 3, 4, 13, 14, 15, 16, 17), die Einschnittfolie oder das Band gemäß der MHC-Dichtigkeitsprüfung mit einer Rillentiefe von 75 Mikrometern leckdicht ist.

3. Drapierprodukt (1, 2, 3, 4, 13, 14, 15, 16, 17) mit einer Klebekante, einer Einschnittfolie oder einem Band nach Anspruch 2,
**dadurch gekennzeichnet, dass** das Drapierprodukt (1, 2, 3, 4, 13, 14, 15, 16, 17), die Einschnittfolie oder das Band gemäß der MHC-Dichtigkeitsprüfung mit einer Rillentiefe von 150 Mikrometern leckdicht ist.

4. Drapierprodukt (1, 2, 3, 4, 13, 14, 15, 16, 17) mit einer Klebekante, einer Einschnittfolie oder einem Band nach Anspruch 3,
**dadurch gekennzeichnet, dass** das Drapierprodukt (1, 2, 3, 4, 13, 14, 15, 16, 17), die Einschnittfolie oder das Band gemäß der MHC-Dichtigkeitsprüfung mit einer Rillentiefe von 200 Mikrometern leckdicht ist.

5. Drapierprodukt (1, 2, 3, 4, 13, 14, 15, 16, 17) mit einer Klebekante, einer Einschnittfolie oder einem Band nach Anspruch 1, wobei das Klebemittel des Drapierprodukts (1, 2, 3, 4, 13, 14, 15, 16, 17) an einer aus einer Kante des Produkts herausragenden Kunststofffolie befestigt ist,
**dadurch gekennzeichnet, dass** das Klebemittel ein Gewicht per Flächeneinheit von 50 g/m² oder mehr aufweist.

6. Drapierprodukt (1, 2, 3, 4, 13, 14, 15, 16, 17) mit einer Klebekante, einer Einschnittfolie oder einem Band nach Anspruch 1, 2 oder 5,
**dadurch gekennzeichnet, dass** das Klebemittel ein Gewicht per Flächeneinheit von 80 g/m² oder mehr aufweist.

7. Drapierprodukt (1, 2, 3, 4, 13, 14, 15, 16, 17) mit einer Klebekante, einer Einschnittfolie oder einem Band nach Anspruch 1,
**dadurch gekennzeichnet, dass** das Klebemittel ein Gewicht per Flächeneinheit von 200 g/m² oder mehr aufweist.

8. Drapierprodukt (1, 2, 3, 4, 13, 14, 15, 16, 17) mit einer Klebekante, einer Einschnittfolie oder einem Band nach einem oder anderen der Ansprüche 1-7, **dadurch gekennzeichnet, dass** das Klebemittel eine Weichheit größer als 12 mm aufweist.

9. Drapierprodukt (1, 2, 3, 4, 13, 14, 15, 16, 17) mit einer Klebekante, einer Einschnittfolie oder einem Band nach einem oder anderen der Ansprüche 1-7, **dadurch gekennzeichnet, dass** das Klebemittel eine Weichheit größer als 14 mm aufweist.

10. Drapierprodukt (1, 2, 3, 4, 13, 14, 15, 16, 17) mit einer Klebekante, einer Einschnittfolie oder einem Band nach einem oder anderen der Ansprüche 1-9, **dadurch gekennzeichnet, dass** das Klebemittel aus einem Heißkleber besteht.

11. Drapierprodukt (1, 2, 3, 4, 13, 14, 15, 16, 17) mit einer Klebekante, einer Einschnittfolie oder einem Band nach einem oder anderen der Ansprüche 1-9, **dadurch gekennzeichnet, dass** das Klebemittel aus einem Siliziumelastomer besteht.

## Revendications

1. Produit drapant (1, 2, 3, 4, 13, 14, 15, 16, 17) avec un bord adhésif, un film d'incision ou une bande, pourvu d'un revêtement (12) d'un adhésif souple respectueux de la peau, **caractérisé en ce que** l'adhésif souple respectueux de la peau présente une douceur supérieure à 10 mm mesurée au moyen du procédé modifié de mesure de la douceur, tel que décrit dans la description, et le produit drapant avec un bord adhésif, le film d'incision ou la bande, pourvu d'un revêtement (12) d'un adhésif souple respectueux de la peau, est étanche aux fuites conformément au test de fuite MHC avec une profondeur d'encoche de 50 micromètres, le procédé dudit test de fuite MHC étant décrit dans la description.

2. Produit drapant (1, 2, 3, 4, 13, 14, 15, 16, 17) avec un bord adhésif, un film d'incision ou une bande selon la revendication 1, **caractérisé en ce que** le produit drapant (1, 2, 3, 4, 13, 14, 15, 16, 17), le film d'incision ou la bande est étanche aux fuites conformément au test de fuite MHC avec une profondeur d'encoche de 75 micromètres.

3. Produit drapant (1, 2, 3, 4, 13, 14, 15, 16, 17) avec un bord adhésif, un film d'incision ou une bande selon la revendication 2, **caractérisé en ce que** le produit drapant (1, 2, 3, 4, 13, 14, 15, 16, 17), le film d'incision ou la bande est étanche aux fuites conformément au test de fuite MHC avec une profondeur d'encoche de 150 micromètres.

4. Produit drapant (1, 2, 3, 4, 13, 14, 15, 16, 17) avec un bord adhésif, un film d'incision ou une bande selon la revendication 3, **caractérisé en ce que** le produit drapant (1, 2, 3, 4, 13, 14, 15, 16, 17), le film d'incision ou la bande est étanche aux fuites conformément au test de fuite MHC avec une profondeur d'encoche de 200 micromètres.

5. Produit drapant (1, 2, 3, 4, 13, 14, 15, 16, 17) avec un bord adhésif, un film d'incision ou une bande selon la revendication 1, en liaison avec lequel l'adhésif du produit drapant (1, 2, 3, 4, 13, 14, 15, 16, 17) est attaché à un film plastique faisant saillie à partir d'un bord du produit, **caractérisé en ce que** l'adhésif a un poids par unité de surface de 50 g/m² ou plus.

6. Produit drapant (1, 2, 3, 4, 13, 14, 15, 16, 17) avec un bord adhésif, un film d'incision ou une bande selon les revendications 1, 2 ou 5, **caractérisé en ce que** l'adhésif a un poids par unité de surface de 80 g/m² ou plus.

7. Produit drapant (1, 2, 3, 4, 13, 14, 15, 16, 17) avec un bord adhésif, un film d'incision ou une bande selon la revendication 1, **caractérisé en ce que** l'adhésif a un poids par unité de surface de 200 g/m² ou plus.

8. Produit drapant (1, 2, 3, 4, 13, 14, 15, 16, 17) avec un bord adhésif, un film d'incision ou une bande selon l'une quelconque des revendications 1 à 7, **caractérisé en ce que** l'adhésif a une souplesse supérieure à 12 mm.

9. Produit drapant (1, 2, 3, 4, 13, 14, 15, 16, 17) avec un bord adhésif, un film d'incision ou une bande selon l'une quelconque des revendications 1 à 7, **caractérisé en ce que** l'adhésif a une souplesse supérieure à 14 mm.

10. Produit drapant (1, 2, 3, 4, 13, 14, 15, 16, 17) avec un bord adhésif, un film d'incision ou une bande selon l'une quelconque des revendications 1 à 9, **caractérisé en ce que** l'adhésif est constitué d'un adhésif thermofusible.

11. Produit drapant (1, 2, 3, 4, 13, 14, 15, 16, 17) avec un bord adhésif, un film d'incision ou une bande selon l'une quelconque des revendications 1 à 9, **caractérisé en ce que** l'adhésif est constitué d'un élastomère de silicone.
